(19) Europäisches Patentamt
European Patent Office
Office européen des brevets

(11) Numéro de publication: **0 004 496**
**B1**

(12) **FASCICULE DE BREVET EUROPEEN**

(21) Numéro de dépôt: **79400151.1**

(22) Date de dépôt: **08.03.79**

(51) Int. Cl.³: **C 07 C 53/48**, C 07 C 51/58, C 07 C 53/16, C 07 C 51/04

(54) **Fabrication de chlorure de monochloracétyle et, éventuellement, d'acide monochloracétique par hydratation de trichloréthylène**

(30) Priorité: **22.03.78 FR 7808264**

(43) Date de publication de la demande: **03.10.79 Bulletin 79/20**

(45) Mention de la délivrance du brevet: **29.10.80 Bulletin 80/22**

(84) Etats contractants désignés: **BE CH DE GB IT NL SE**

(56) Documents cités: **Fr - A - 1 488 064**

(73) Titulaire: **RHONE-POULENC INDUSTRIES**
**22, avenue Montaigne**
**F - 75008 Paris (FR)**

(72) Inventeur: **Correia, Yves**
**Les Lauzières**
**F - 04160 Chateau Arnoux (FR)**
**Dumas, Gérard**
**3, rue Jacques Level**
**F - 04600 Saint-Auban (FR)**

(74) Mandataire: **Rosenberg, Roger et al**
**RHONE POULENC Service Brevets Chimie et Polymères B.P. 753**
**F - 75360 Paris Cedex 08 (FR)**

**EP 0 004 496 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Fabrication de chlorure de monochloracétyle et, éventuellement, d'acide monochloracétique par hydratation de trichloréthyléne

La présente invention concerne un procédé de fabrication de chlorure de monochloracétyle éventuellement accompagné d'acide monochloracétique, par hydration de trichloréthylène et/ou de tétrachloro-1,1,1,2 éthane, en présence d'un catalyseur au chlorure de fer.

Il est connu de préparer de l'acide monochloracétique suivant le brevet US 1.304.108 par la réaction d'hydratation de trichloréthylène à 150—200°C, en présence d'acide sulfurique ayant une concentration d'au moins 95% ou d'oléum, comme agent d'hydratation.

Les auteurs de ce brevet se basent sur la mise en jeu de réactions hypothétiques pour expliquer la formation d'acide monochloracétique, ces réactions étant les suivantes:

$$SO_4H_2 + CCl_2 - CHCl \rightarrow SO_2(OH) - O - CCl_2 - CH_2Cl$$

$$SO_2(OH) - O - CCl_2 - CH_2Cl + H_2O \rightarrow SO_4H_2 + CCl_2(OH) — CH_2Cl$$

$$CCl_2(OH) - CH_2Cl \rightarrow CH_2Cl - COCl + HCl$$

$$CH_2Cl - COCl + H_2O \rightarrow CH_2Cl - COOH + HCl$$

ce qui, globalement, fait apparaitre comme si l'eau seule produit la transformation:

$$CCl_2 = CHCl + 2H_2O \rightarrow CH_2Cl - COOH + 2HCl$$

Toutefois, la formation intermédiaire de chlorure de monochloracétyle n'a pas été démontrée ou mise en évidence, ce qui ressort clairement du brevet français n° 516367 qui indique l'obtention d'acide monochloracétique exclusivement, en faisant réagir de l'eau et du trichloréthylène en présence d'acide sulfurique à 90% à 190°C.

Le brevet suisse n° 86192 décrit spécifiquement l'obtention de chlorure de monochloracétyle à partir de trichloréthylène également par chauffage vers 110°C avec de l'acide sulfurique mais à 1'état anhydre (100%). Il ne peut donc s'agir de réaction d'hydratation de trichloréthylène dans ce procédé.

Plus récemment, le brevet US 3.742.047 a décrit un procédé d'obtention de chlorure de monochloracétyle en faisant réagir un mélange de trichloréthylène et d'acide monochloracétique avec un dérivé d'acide sulfonique ou d'acide sulfurique à 75—125°C. L'acide utilisé dans cette réaction est essentiellement exempt d'eau tel que l'acide sulfurique à 100% ou les acides toluènesulfonique, méthanesulfonique, éthanesulfonique et chlorosulfonique. Dans le cas de l'acide sulfurique à 100% l'équation globale de la réaction s'écrit:

$$CHCl = CCl_2 + Cl—CH_2—COOH + H_2SO_4 \rightarrow 2ClCH_2 - COCl + H_2SO_4$$

Cependant, ce procédé consomme de l'acide monochloracétique alors qu'en lui-même c'est un produit de départ utile pour de nombreuses synthèses, par exemple celles de composés doués de propriétés herbicides ou parasiticides renfermant le groupe chloro-acétyle. En outre, la conduite de la réaction exige un contrôle rigoureux de la température qui ne doit pas dépasser 125°C car, au-dessus, il se forme des sous-produits de polymérisation, de sulfonation et de déshydratation (anhydrides).

Suivant le brevet français 2.070.428, cette dernière réaction est réalisable sous pression de chlorure d'hydrogène, mais en metant un oeuvre du chlorure fernique comme catalyseur au lieu d'acide sulfurique ou sulfonique. Cependant, ce procédé consomme de l'acide monochloracétique comme le précédent. De plus, pour un fonctionnement en continu du procédé, il est nécessaire d'introduire sous pression l'acide monochloracétique à l'état fondu, le catalyseur anhydre sous forme solide, alors que le trichloréthylène est en phase liquide. Ces opérations posent des problèmes techniques difficiles à résoudre.

Le procédé décrit dans le brevet français 2.070.427 qui ne diffère du brevet français précédent que par le réactif de départ lequel est du tétrachloro-1,1,1,2 éthane, au lieu de trichloréthylène, présente les mêmes difficultés techniques faisant obstacles à une réalisation aisée à l'échelle de fabrication industrielle.

La présente invention, a pour but de remédier aux inconvénients précités par la mise en oeuvre d'un procédé simple de fabrication en continu, de chlorure de monochloracétyle, accompagné, le cas échéant, d'acide monochloracétique par l'action directe de l'eau sur le trichloréthylène suivant la réaction:

$$CHCl = CCl_2 + H_2O \rightarrow ClCH_2 - COCl + HCl$$

Conformément à l'invention, on fait réagir de l'eau avec du trichloréthylène et/ou du tétrachloro-1,1,1,2 éthane, cette réaction d'hydratation étant effectuée en phase liquide en présence de chlorure

ferrique, partiellement en suspension, et sous pression de chlorure d'hydrogène, à une température comprise entre 80 et 180°C.

La pression de chlorure d'hydrogène pour réaliser le procédé de l'invention est comprise entre 5 et 80 bars absolus, plus avantageusement entre 15 et 60 bars absolus, et, de préférence, de 20 à 40 bars absolus.

La demanderesse a constaté qu'à chaque valeur de pression de travail donnée, pris dans l'intervalle précité, la vitesse de réaction prend une valeur maximum pour une température spécifique, ce qui permet de définir un intervalle préféré de température encadrant cette température spécifique pour la pression de travail choisie. Par exemple, lorsqu'on opère à 30 bars absolus, on observe qu'aux températures en dessous de 100°C et à celles au-dessus de 180°C, la réaction est pratiquement négligeable, et la vitesse de réaction commence à décroitre à partir de 160°C environ, si bien que l'intervalle préféré de température de réaction se situe entre 140 et 170°C lorsqu'on choisit d'opérer à environ 30 bars absolus.

Selon l'invention, le rapport molaire des réactifs trichloréthylène et/ou tétrachloro-1,1,1,2 éthane d'alimentation/eau est, en général, d'au moins 0,6. On observe que la quantité d'acide monochloracétique accompagnant le chlorure de monochloracétyle est d'autant plus forte que le rapport molaire trichloréthylène et/ou tétrachloro-1,1,1,2 éthane d'alimentation/eau se rapproche de 0,6. Par contre, pour des valeurs de ce rapport molaire au-delà de 2, les quantités de produits secondaires indésirés tels que pentachloréthane et perchloréthylène, provenant de la chloration additive de trichloréthylène par $FeCl_3$ et réduction concomitante d'une partie de $FeCl_3$ en $FeCl_2$, et de pentachlorobutadiène provenant de la dimérisation de trichloréthylène, deviennent non-négligeables, voire importantes. Si bien que l'on peut très bien opérer à des rapports molaires de 2,5 à 3 par exemple si ces produits secondaires sont souhaités.

Dans le cas où l'on obtention de chlorure de monochloracétyle avec une très bonne sélectivité, on a intérêt à utiliser des rapports molaires trichloréthylène et/ou tétrachloro-1,1,1,2 éthane d'alimentation/eau compris entre 1,2 et 1,8.

Lorsqu'on désire obtenir simultanément et essentiellement du chlorure de monochloracétyle et de l'acide monochloracétique, le rapport molaire trichloréthylène et/ou tétrachloro-1,1,1,2 éthane d'alimentation/eau est choisi entre 0,6 et 1,2.

La quantité de chlorure ferrique que l'on introduit dans le milieu réactionnel est comprise entre 0,1 et 15% en poids du milieu réactionnel, hors le chlorure d'hydrogène aussi bien gazeux que celui qui y est dissous. En-dessous de 0,1% en poids de $FeCl_3$, la vitesse de réaction est excessivement faible, alors qu'au-dessus de 15% en poids, la séparation et la récupération ultérieure du catalyseur se trouvant dans le milieu réactionnel se compliquent. Dans le cas où il se forme du chlorure ferreux à la suite des réactions indésirées signalées plus haut, le catalyseur peut être séparé du milieu réactionnel par tout moyen connu et le chlorure ferreux peut être oxydé à nouveau en $FeCl_3$ par du chlore gazeux ou un agent susceptible de libérer du chlore actif comme l'eau de chlore, l'eau de javel, ou le bioxyde de chlore ou en général, par tout oxydant de l'HCl. Le catalyseur ainsi régénéré peut alors être recyclé au milieu réactionnel.

Selon une forme particulièrement avantageuse de réalisation du procédé de l'invention, le chlorure ferrique est introduit dans le milieu réactionnel sous la forme d'une solution aqueuse.

Dans un mode de récupération du catalyseur dans le cadre du procédé de l'invention, on soumet l'effluent de la zone de réaction à un fractionnement pour séparer le chlorure de monochloracétyle et les hydrocarbures chlorés n'ayant pas réagi, du mélange renfermant les chlorures ferreux et ferrique. Ce mélange est traité à l'eau et réoxydé par exemple par du chlore, et la solution ferrique qui en résulte est ensuite recyclée à la zone de réaction.

Le temps de passage des réactifs dans la zone de réaction calculé sur les débits d'entrée, est généralement de 1 à 8 heures, et de préférence de 2 à 6 heures.

Le trichloréthylène de départ peut, selon l'invention, provenir de tétrachloro-1,1,1,2 éthane lequel dans les conditions de la réaction d'hydratation de l'invention, subit une déshydrochloration au moins partielle, in situ, en trichloréthylène établissant ainsi un équilibre qui dépend de la température et de la pression mises en oeuvre, entre le tétrachloro-1,1,1,2 éthane et le trichloréthylène. Si bien qu'on peut utiliser comme produit de départ, selon l'invention, du trichloréthylène et/ou du tétrachloro-1,1,1,2 éthane, ce qu'exprime le rapport molaire des réactifs trichloréthylène et/ou tétrachloro-1,1,1,2 éthane/eau.

Ainsi, dans le cadre d'un procédé en continu, le tétrachloro-1,1,1,2 éthane au même titre que l'acide monochloracétique, est un sous-produit utile qui peut être recyclé avec le trichloréthylène de l'alimentation. De son côté, l'acide monochloracétique éventuellement formé, peut si on le désire, être recyclé avantageusement en mélange avec le chlorure ferrique surtout lorsque celui-ci est utilisé sous forme d'une solution aqueuse. Dans ce cas, le quantité de chlorure ferrique est supérieure à 20% en poids par rapport à la quantité présente d'acide monochloracétique dans le milieu réactionnel et peut dépasser jusqu'à 10 fois le poids de cet acide présent.

Les exemples suivants ont pour seul but d'illustrer le procédé de la présente invention et ils ne doivent pas être considérés comme limitatifs.

Dans ces exemples, le taux de conversion de trichloréthylène et/ou de tétrachloro-1,1,1,2 éthane utilisé, suivant le cas, comme produit de départ, est défini par le rapport:

$$\frac{\text{nombre de moles de } (CCl_2 = CHCl + CCl_3CH_2Cl) \text{ transformés}}{\text{nombre de moles de } (CCl_2 = CHCl + CCl_3CH_2Cl) \text{ alimentés au réacteur}} \times 100$$

avec le numérateur égal à la différence entre le nombre de moles de ($CCl_2 = CHCl + CCl_3CH_2Cl$) alimentés au réacteur et le nombre de moles de ($CCl_2 = CHCl + CCl_3CH_2Cl$) sortis du réacteur.

La sélectivité en $ClCH_2$—COCl est définie par le rapport:

$$\frac{\text{nombre de moles de } ClCH_2\text{—}COCl \text{ produit}}{\text{nombre de moles de } (CCl_2 = CHCl + CCl_3CH_2Cl) \text{ transformés}} \times 100$$

La sélectivité en $ClCH_2$—COOH est définie par le rapport:

$$\frac{\text{nombre de moles de } ClCH_2\text{—}COOH \text{ produit}}{\text{nombre de moles de } (CCl_2 = CHCl + CCl_3\text{—}CHCl \text{ transformés}} \times 100$$

EXEMPLE 1

Dans un réacteur en acier verré, agité, équipé de régulations de pression et de niveau, on introduit en continu 1264 kg/h (9,61 kmoles) de trichloréthylène et 181 kg/h d'une solution aqueuse de $FeCl_3$ contenant 40,3% de $FeCl_3$ (0,45 kmole de $FeCl_3$ et 6 kmoles d'$H_2O$).

Le rapport molaire trichloréthylène/eau est de 1,6 et la quantité de $FeCl_3$ représente 5,8% en poids du milieu réactionnel hors l'HCl (aussi bien gazeux que celui qui y est dissous).

La pression régnant dans le réacteur est maintenue à 30 bars absolus et la température est fixée à 150°C. Le temps de passage des réactifs est de 4 heures. On soutire en continu du réacteur 1257 kg/h de liquide hors HCl dissous renfermant:

— 656,5 kg de chlorure de monochloracétyle,
— 9,5 kg d'acide monochloracétique
— 366 kg de trichloréthylène
— 141 kg de tétrachloro-1,1,1,2 éthane
— 16 kg de produits lourds chlorés (pt d'eb>150°C)
— 49 kg de $FeCl_3$
— 19 kg de $FeCl_2$

On récupère au total 5,16 kmoles/h d'HCl correspondant à l'HCl soutiré en continu en phase gazeuse et à l'HCl dissous dégagé par la détente ultérieure de l'effluent liquide.

Afin de récupérer le catalyseur, cet effluent est fractionné pour séparer le chlorure de monochloracétyle et les hydrocarbures chlorés n'ayant pas réagi, du mélange comprenant les chlorures ferreux et ferrique. Ce mélange est traité à l'eau et réoxydé par le chlore pour donner une solution ferrique avant d'être recyclé au réacteur.

Le taux de conversion, les sélectivités en $ClCH_2$—COCl et en $ClCH_2$—COOH tels que définis ci-dessus sont respectivement de 62,3%, 97,0% et 1,7%.

EXEMPLE 2

En suivant les mêmes étapes du processus et dans les mêmes conditions de température et de pression de chlorure d'hydrogène que l'exemple 1, on introduit en continu dans ce réacteur 1151 kg/h (8,75 kmoles) de trichloréthylène et 266,5 kg/h d'une solution aqueuse de $FeCl_3$ contenant 27,3% en poids de $FeCl_3$ (10,75 kmoles d'$H_2O$ et 0,45 kmoles/m3 de $FeCl_3$).

Le rapport molaire trichloréthylène/eau est de 0,81. La quantité de $FeCl_3$ représente 7,1% en poids du milieu réactionnel hors HCl (aussi bien dissous que gazeux).

Le débit d'entrée des réactifs est de 1 m3/h. Le temps de passage des réactifs dans le réacteur est de 4 heures. On soutire du réacteur 1032 kg/h de produit réactionnel hors HCl dissous contenant:

— 430,5 kg de chlorure de monochloracétyle
— 328 kg d'acide monochloracétique
— 167 kg de trichloréthylène
— 32 kg de tétrachloro-1,1,1,2 éthane
— 2 kg de produits lourds chlorés (pt. d'éb.>150°C)
— 70 kg de $FeCl_3$
— 2,6 kg de $FeCl_2$
— 10,56 kmoles/h d'HCl sont récupérées.

Le taux de conversion, les sélectivités en $CH_2Cl—COCl$ et en $CH_2Cl—COOH$ tels que définis plus haut, sont respectivement de 83,3% 52,3% et 47,6%.

## EXEMPLE 3

En suivant les mêmes étapes du processus et dans les mêmes conditions de température, de temps de passage et de pression de chlorure d'hydrogène que l'exemple 1, on introduit en continu dans ce réacteur:

— 1249,5 kg/h (9,5 kmoles) de trichloréthylène
— 190,5 kg/h (7,6 kmoles $H_2O$ et 0,33 kmoles $FeCl_3$) de

solution aqueuse de $FeCl_3$ contenant 28,1% en poids de $FeCl_3$.

Le rapport molaire trichloréthylène/eau est de 1,25. La quantité de $FeCl_3$ représente 4,5% en poids du milieu réactionnel hors de chlorure d'hydrogène qui y est dissous aussi bien que celui à l'état gazeux.

Le débit d'entrée des réactifs est de 1 m3/h.

On soutire du réacteur 1188,5 kg/h de produit réactionnel hors HCl dissous contenant:

— 447 kg de chlorure de monochloracétyle
— 172 kg d'acide monochloracétique
— 393 kg de trichloréthylène
— 119 kg de tétrachloro-1,1,1,2 éthane
— 6 kg de produits lourds chlorés (pt. d'éb.>150°C)
— 45,5 kg de $FeCl_3$
— 6 kg de $FeCl_2$
— 6,89 kmoles/ d'HCl récupéré

Le taux de conversion, les sélectivités en $CH_2Cl—COCl$ et en $CH_2Cl—COOH$ tels que définis plus haut sont respectivement de 61,1% 68,3% et 31,4%.

## EXEMPLE 4

En effectuant les mêmes étapes et dans les mêmes conditions de température et de pression d'HCl que l'exemple 1, on introduit en continu dans ce réacteur:

— 1663,5 kg/h (12,65 kmoles) de trichloréthylène
— 260,5 kg/h (9,05 kmoles $H_2O$ et 0,6 kmole $FeCl_3$) de solution aqueuse à 37,4% en poids de $FeCl_3$.

Le rapport molaire trichloréthylène/eau est de 1,4. Le débit d'entrée des réactifs est de 1,33 m3/h. Le temps de passage des réactifs dans le réacteur est de 3 heures. La quantité de $FeCl_3$ représente 5,9% en poids par rapport an milieu réactionnel hors l'HCl (aussi bien celui à l'état gazeux que celui à l'état dissous).

On soutire alors du réacteur 1649 kg/h de produit réactionnel hors HCl dissous contenant:

— 545 kg de chlorure de monochloracétyle
— 200 kg d'acide monochloracétique
— 539 kg de trichloréthylène
— 254 kg de tétrachloro-1,1,1,2 éthane
— 24 kg de produits lourds chlorés (pt. d'éb.>150°C)
— 57 kg de $FeCl_3$
— 30 kg de $FeCl_2$
— 7,54 kmoles/h d'HCl récupéré

Le taux de conversion, les sélectivités en $CH_2Cl—COCl$ et en $CH_2Cl—COOH$ tels que définis plus haut, sont respectivement de 55,7% 68,5% et 30,1%.

EXEMPLE 5

Dans les mêmes conditions de température, de pression d'HCl et en effectuant les mêmes étapes du processus que dans l'exemple 1, on introduit en continu dans le réacteur:

— 1097 kg/h de trichloréthylène
— 196 kg/h de tétrachloro-1,1,1,2 éthane recyclé provenant de l'effluent dudit réacteur
— 110 kg/h d'eau
— 90 kg/h d'acide monochloracétique recyclé provenant également de l'effluent dudit réacteur
— 66 kg/h de $FeCl_3$ dont 95% provient du recyclage du catalyseur récupéré de l'effluent dudit réacteur

Le rapport molaire ($CHCl=CCl_2$ + $CCl_3—CH_2Cl$)/$H_2O$ est 1,56. Le débit d'entrée dans le réacteur des constituants du mélange réactionnel est d'environ 1 m3/h et leur temps de passage est de 4 heures. La quantité de $FeCl_3$ représente 4,9% en poids du milieu réactionnel hors l'HCl (aussi bien dissous que gazeux).

On soutire du réacteur 1336 kg/h de mélange réactionnel hors HCl dissous contenant:

— 690,5 kg de chlorure de monochloracétyle
— 90 kg d'acide monochloracétique
— 284 kg de trichloréthylène
— 196 kg de tétrachloro-1,1,1,2 éthane
— 14,5 kg de produits lourds chlorés (pt. d'éb.>150°C)
— 45 kg de $FeCl_3$
— 16 kg de $FeCl_2$
— 6,11 kmoles/h d'HCl récupéré

Le taux de conversion du trichloréthylène et du tétrachloro-1,1,1,2 éthane alimentés et la sélectivité en $ClCH_2—COCl$ tels que définis plus haut, sont respectivement de 65% et 98,9%.

EXEMPLE 6

Dans les mêmes conditions de température, de pression d'HCl et en effectuant les mêmes opérations que dans l'exemple 1, on introduit en continu dans le réacteur:

— 2125 kg/h de tétrachloro-1,1,1,2 éthane
— 260,5 kg/h (9,05 kmoles $H_2O$ et 0,6 kmole $FeCl_3$) de solution aqueuse à 37,4% en poids de $FeCl_3$

Le rapport molaire tétrachloro-1,1,1,2 éthane/eau est de 1,4. Le débit d'entrée des réactifs est de 1,49 m3/h.

Le temps de passage des réactifs dans le réacteur est de 2,7 heures. La quantité de $FeCl_3$ représente 6% en poids par rapport au milieu réactionnel hors HCl (aussi bien dissous que gazeux).

On soutire alors due réacteur 1632,5 kg/h de produit réactionnel hors HCl dissous contenant:

— 864,5 kg de chlorure de monochloracétyle
— 65 kg d'acide monochloracétique
— 393 kg de trichloroéthylène
— 201,5 kg de tétrachloro-1,1,1,2 éthane
— 18 kg de produits lourds (pt. d'éb.>150°C)
— 25,5 kg de $FeCl_2$
— 65 kg de $FeCl_3$
— 20,6 kmoles/h d'HCl récupéré

Le taux de conversion du tétrachloro-1,1,1,2 éthane et les sélectivités en $CH_2Cl—COCl$ et en $CH_2Cl—COOH$ sont respectivement de 66.9%, 90,4% et 8,2%.

**Revendications**

1. Procédé continu de fabrication de chlorure de monochloracétyle le cas échéant accompagné d'acide monochloracétique, à partir de trichloréthylène et/ou de tétrachloro-1,1,1,2 éthane, caractérisé par le fait que l'on effectue une réaction d'hydration du trichloréthylène et/ou du tétrachloro-1,1,1,2 éthane sous pression de chlorure d'hydrogène en phase liquide en présence de chlorure ferrique partiellement en suspension.

2. Procédé selon la revendication 1, caractérisé par le fait que l'hydration est effectuée à une température comprise entre 80 et 180°C, et, de préférence, entre 140 et 170°C.

3. Procédé selon la revendication 1 ou 2, caractérisé par le fait que le chlorure ferrique est introduit dans le milieu réactionnel sous forme d'une solution aqueuse.

4. Procédé selon l'une quelconque des revendications 1 à 3, caractérisé par le fait que le trichloréthylène et/ou le tétrachloro-1,1,1,2 éthane et l'eau sont introduits dans la zone de réaction dans un rapport molaire supérieur à 0,6.

5. Procédé selon l'une quelconque des revendications 1 à 4, caractérisé par le fait que le trichloréthylène et/ou le tétrachloro-1,1,1,2 éthane et l'eau sont dans un rapport molaire compris entre 1,2 et 1,8.

6. Procédé selon l'une quelconque des revendications 1 à 5, caractérisé par le fait que le trichloréthylène à l'alimen tation provient en partie ou en totalité de la déshydrochloration in situ de tétrachloro-1,1,1,2 éthane.

7. Procédé selon l'une quelconque des revendications 1 à 6, caractérisé par le fait que l'acide monochloracétique éventuellement formé est recyclé au milieu réactionnel.

8. Procédé selon l'une quelconque des revendications 1 à 7, caractérisé en ce que la quantité de $FeCl_3$ est comprise entre 0,1 et 15% en poids de l'ensemble du milieu réactionnel, hors le chlorure d'hydrogène aussi bien à l'état gazeux que celui qui y est dissous.

9. Procédé selon les revendications 3 et 7, caractérisé en ce que l'acide monochloracétique éventuellement formé est recyclé avantageusement en mélange avec la solution de chlorure ferrique.

10. Procédé selon la revendication 9, caractérisé par le fait que la quantité de chlorure ferrique est supérieure à 20% en poids par rapport à la quantité présente d'acide monochloracétique dans le milieu réactionnel.

11. Procédé selon l'une quelconque des revendications 1 à 10 caractérisé par le fait que la pression de chlorure d'hydrogène est de 5 à 80 bars absolus, et de préférence, de 20 à 40 bars absolus.

12. Procédé selon l'une quelconque des revendications 1 à 11, caractérisé par le fait que dans le cas où le chlorure ferrique de milieu réactionnel donne du chlorure ferreux par suite de réactions secondaires, celui-ci est réoxydé par du chlore actif en $FeCl_3$.

13. Procédé selon la revendication 12, caractérisé par le fait que la réoxydation du chlorure ferreux est effectuée après que l'on soumet le milieu réactionnel effluent à un fractionnement pour séparer le chlorure de monochloracétyle et les hydrocarbures chlorés n'ayant pas réagi, du mélange renfermant les chlorures ferreux et ferrique.

**Patentansprüche**

1. Kontinuierliches Verfahren zur Herstellung von Monochloracetylchlorid, gegebenenfalls zusammen mit Monochloressigsäure, aus Trichloräthylen und/oder aus 1,1,1,2-Tetrachloräthan, dadurch gekennzeichnet, daß Trichloräthylen und/oder 1,1,1,2-Tetrachloräthan unter Druck von Chlorwasserstoff in flüssiger Phase in Gegenwart von teilweise suspendiertem Eisen(III)-Chlorid hydratisiert wird.

2. Verfahren nach Anspruch 1, dadurch gekennzeichnet, daß die Hydratisierung bei einer Temperatur von 80° bis 180°C, vorzugsweise von 140° bis 170°C, durchgeführt wird.

3. Verfahren nach Anspruch 1 oder 2, dadurch gekennzeichnet, daß das Eisen(III)-chlorid in Form einer wässerigen Lösung in die Reaktionszone eingeführt wird.

4. Verfahren nach einem der Ansprüche 1 bis 3, dadurch gekennzeichnet, daß Trichloräthylen und/oder 1,1,1,2-Tetrachloräthan und Wasser in die Reaktionszone mit einem Molverhältnis über 0,6 eingebracht werden.

5. Verfahren nach einem der Ansprüche 1 bis 4, dadurch gekennzeichnet, daß Trichloräthylen und/oder 1,1,1,2-Tetrachloräthan und Wasser in einem Molverhältnis von 1,2 bis 1,8 stehen.

6. Verfahren nach einem der Ansprüche 1 bis 5, dadurch gekennzeichnet, daß das zugeführte Trichloräthylen ganz oder teilweise von der In-situ-Dehydrochlorierung von 1,1,1,2-Tetrachloräthan herkommt.

7. Verfahren nach einem der Ansprüche 1 bis 6, dadurch gekennzeichnet, daß die gegebenenfalls entstandene Monochloressigsaüre in das Reaktionsmedium zurückgeführt wird.

8. Verfahren nach einem der Ansprüche 1 bis 7, dadurch gekennzeichnet, daß der Anteil von $FeCl_3$ zwischen 0,1 und 15 Gew.% bezogen auf das gesamte Reaktionsmedium mit Ausnahme des gasförmigen und gelösten Chlorwasserstoffs, liegt.

9. Verfahren nach den Ansprüchen 3 und 7, dadurch gekennzeichnet, daß die gegebenenfalls entstandene Monochloressigsaüre vorteilhaft zusammengemischt mit der Eisen(III)-chlorid-Lösung zurückgeführt wird.

10. Verfahren nach Anspruch 9, dadurch gekennzeichnet, daß die Menge des Eisen(III)-chlorids über 20 Gew.%, bezogen auf die im Reaktionsmedium vorhandene Menge der Monochloressigsäure, liegt.

11. Verfahren nach einem der Ansprüche 1 bis 10, dadurch gekennzeichnet, daß der Druck des Chlorwasserstoffs zwischen 5 und 80 ata, vorzugsweise zwischen 20 und 40 ata, liegt.

12. Verfahren nach einem der Ansprüche 1 bis 11, dadurch gekennzeichnet, daß das Eisen(II)-chlorid, welches gegebenenfalls aus dem Eisen(III)-chlorid aufgrund von Nebenreaktionen im Reaktionsmedium entsteht, durch aktives Chlor zu $FeCl_3$ wieder oxydiert wird.

13. Verfahren nach Anspruch 12, dadurch gekennzeichnet, daß die Wiederoxydierung des Eisen(II)-chlorids erfolgt, nachdem das auslaufende Reaktionsmedium fraktioniert wird, um das Monochloracetylchlorid und die nicht umgesetzten Chlorkohlenwasserstoffe vom Gemisch zu trennen, welches Eisen(II)- und Eisen(III)-chlorid enthält.

**Claims**

1. A continuous process for the production of monochloracetyl chloride, which may be accompanied by monochloracetic acid, from trichloroethylene and/or 1,1,1,2-tetrachloroethane, characterised in that a hydration reaction of trichloroethylene and/or 1,1,1,2-tetrachloroethane is performed under pressure of hydrogen chloride in liquid phase in the presence of ferric chloride partially in suspension.

2. A process according to claim 1, characterised in that hydration is carried out at a temperature of from 80 to 180°C, and preferably from 140 to 170°C.

3. A process according to claim 1 or 2, characterised in that the ferric chloride is introduced into the reaction medium in the form of an aqueous solution.

4. A process according to any one of claims 1 to 3, characterised in that the trichloroethylene and/or 1,1,1,2-tetrachloroethane and water are introduced into the reaction zone in a molar ratio greater than 0.6.

5. A process according to any one of claims 1 to 4, characterised in that the trichloroethylene and/or 1,1,1,2-tetrachloroethane and water are in a molar ratio of from 1.2 to 1.8.

6. A process according to any one of claims 1 to 5, characterised in that the trichloroethylene for the feed originates partially or totally from the in situ dehydrochlorination of 1,1,1,2-tetrachloroethane.

7. A process according to any one of claims 1 to 6, characterised in that the monochloroacetic acid which is possibly formed is recycled to the reaction medium.

8. A process according to any one of claims 1 to 7, characterised in that the amount of $FeCl_3$ is from 0.1 to 15% by weight of the whole of the reaction medium, except for the hydrogen chloride both in the gaseous state and that which is dissolved therein.

9. A process according to claims 3 and 7, characterised in that the monochloroacetic acid which is possibly formed is advantageously recycled in admixture with the ferric chloride solution.

10. A process according to claim 9, characterised in that the amount of ferric chloride is higher than 20% by weight with respect to the amount of monochloroacetic acid present in the reaction medium.

11. A process according to any of claims 1 to 10 characterised in that the hydrogen chloride pressure is from 5 to 80 bars absolute, and preferably from 20 to 40 bars absolute.

12. A process according to any one of claims 1 to 11, characterised in that, in the case where the ferric chloride of the reaction medium gives ferrous chloride as a consequence of secondary reactions, the ferrous chloride is re-oxidised by active chlorine to $FeCl_3$.

13. A process according to claim 12, characterised in that re-oxidation of the ferrous chloride is carried out after the reaction medium effluent is subjected to a fractionating operation to separate the unreacted chlorinated hydrocarbons and monochloracetyl chloride from the mixture containing the ferrous and ferric chloride.